Europäisches Patentamt

(19) European Patent Office

Office européen des brevets

(11) EP 0 559 212 B1

## (12) EUROPEAN PATENT SPECIFICATION

(45) Date of publication and mention
of the grant of the patent:
**20.08.1997 Bulletin 1997/34**

(51) Int. Cl.$^6$: **C07C 68/00**, C07C 69/96

(21) Application number: **93103567.9**

(22) Date of filing: **05.03.1993**

(54) **Process for preparing carbonic diester**

Verfahren zur Herstellung eines Diesters der Kohlensäure

Procédé de préparation d'un diester d'acide carbonique

(84) Designated Contracting States:
**DE FR GB IT**

(30) Priority: **06.03.1992 JP 97506/92
30.06.1992 JP 210601/92
31.07.1992 JP 245411/92**

(43) Date of publication of application:
**08.09.1993 Bulletin 1993/36**

(73) Proprietor: **UBE INDUSTRIES, LTD.
Ube-shi, Yamaguchi-ken 755 (JP)**

(72) Inventors:
• **Nishihira, Keigo,
c/o Ube Chemical Factory
Ube-shi, Yamaguchi-ken (JP)**
• **Tanaka, Shuji,
c/o Ube Chemical Factory
Ube-shi, Yamaguchi-ken (JP)**

• **Kawashita, Tetsuro,
c/o Ube Chemical Factory
Ube-shi, Yamaguchi-ken (JP)**
• **Nishida, Yuki,
c/o Ube Chemical Factory
Ube-shi, Yamaguchi-ken (JP)**
• **Matsuzaki, Tokuo,
c/o Ube Research Laboratory
Ube-shi, Yamaguchi-ken (JP)**
• **Abe, Koji,
c/o Ube Research Laboratory
Ube-shi, Yamaguchi-ken (JP)**

(74) Representative: **Hansen, Bernd, Dr. Dipl.-Chem.
et al
Hoffmann Eitle,
Patent- und Rechtsanwälte,
Postfach 81 04 20
81904 München (DE)**

(56) References cited:
**EP-A- 0 464 460         EP-A- 0 501 507**

## Description

The present invention relates to a process for preparing a carbonic dialkyl ester or dialkyl carbonate (to be referred to as carbonic diester) such as dimethyl carbonate by reaction of an alkyl nitrite such as methyl nitrite and carbon monoxide by vapor phase contact catalysis in the presence of a solid catalyst having weak acidity, wherein platinum group metal ion is exchanged on an ion exchange zeolite carrier.

Carbonic dialkyl esters such as dimethyl carbonate are extremely useful as raw materials and for synthesis of aromatic polycarbonates and various chemical products, and as solvents.

For example, a process is proposed in Japanese Unexamined Patent Publication No. 181051/1985 for the preparing of dimethyl carbonate by reacting methyl nitrite and carbon monoxide in the vapor phase in the presence of a solid catalyst, wherein a platinum group metal or its compound is carried on a carrier such as activated carbon, and oxidant at 10 mol% or more as oxygen per carbon monoxide. However, in this process, despite an oxidant such as oxygen being present in the above-mentioned proportion with respect to carbon monoxide in order to suppress production of dimethyl oxalate byproduct, a considerable amount of dimethyl oxalate is produced at high selectivity. This results in the problems of decreased selectivity of dimethyl carbonate, reduced reaction rate and low space time yield (STY to be described later). In addition, when reacting the mixed gas consisting of methyl nitrite, carbon monoxide, oxygen and so forth, there is the risk of explosion thus resulting in additional problems in terms of safety during the reaction.

More recently, a process for preparing dimethyl carbonate by vapor phase catalytic reaction of carbon monoxide and methyl nitrite is disclosed in Japanese Unexamined Patent Publication No. 141243/1991 (Japanese Patent Application No. 274816/1989). In this process, a solid catalyst is used wherein a halogen compound such as a chloride of a platinum group metal, and a halogen compound such as the chloride of a secondary component metal selected from the group consisting of iron, copper, cobalt, nickel and tin, are carried on activated carbon and so on.

Moreover, Japanese Unexamined Patent Publication No. 89458/1992 (Japanese Patent Application No. 201146/1990) discloses a modified process wherein a minute amount of hydrogen chloride is added to the reaction system in the above-mentioned preparing of dimethyl carbonate.

EP-A-0 464 460 discloses a process for prepraing a dialkylcarbonate by reacting carbon monoxide and an alkylnitrite in the presence of a modified platinum metal supported catalyst whose carrier may be a zeolite. However, in the above-mentioned preparing processes, the activity of the catalyst gradually decreases due to the releasing (dispersing) of minute amounts of chlorine from the catalyst. Thus, in order to prevent this, it is necessary to add a minute amount of hydrogen chloride to the reaction system.

As a result, said processes have the problems of chlorine ending up contaminating the target product of dimethyl carbonate, and the potential for the reaction apparatus being corroded by the chlorine.

## SUMMARY OF THE INVENTION

The object of the present invention is to provide a process for the preparing of carbonic diester wherein there is no occurrence of problems caused by the presence of chlorine as in the above-mentioned prior art during preparing of carbonic diester from alkyl nitrite and carbon monoxide in the vapor phase in the presence of a solid catalyst, catalyst activity can be maintained at a high level for a long period of time, and dimethyl carbonate can be manufactured at high selectivity (and/or high yield) under mild reaction conditions.

The inventors of the present invention discovered that the problems caused by the presence of chlorine in the above-mentioned prior art can be solved by the formation of carbonic diester by reacting alkyl nitrite with carbon monoxide at a specific high mole ratio using a solid catalyst having weak acidity that is obtained by ion exchange of a platinum group metal ion such as palladium on an ion exchange zeolite carrier, thus leading to completion of the present invention.

In other words, the present invention relates to a process for preparing carbonic diester comprising the step of reacting an alkyl nitrite and carbon monoxide in the vapour phase in the presence of a solid catalyst obtainable by ion exchange of a platinum group metal ion on an ion exchange zeolite carrier, wherein the mole ratio (RONO/CO) of the alkyl nitrite to carbon monoxide is 2 or more, and wherein the solid catalyst has weak acidity which is characterized by:

(i) an acidic strength as measured by the desorption temperature determined by the ammonia-temperature programmed desorption (TPD) method prescribed by the Catalyst Society of less than 360°C, and
(ii) an acidic amount determined by the ammonia-TPD method of 20 or less based upon the acidic amount of an HY type zeolite being 100.

## DESCRIPTION OF THE PREFERRED EXAMPLE

In the present invention, a process for preparing dialkyl carbonate by vapor phase catalytic reaction of alkyl nitrite and carbon monoxide in the presence of a solid catalyst having weak acidity, wherein palladium ion is exchanged on an

ion exchange zeolite carrier, using a raw material gas wherein the mole ratio of alkyl nitrite and carbon monoxide (RONO/CO) is 2-30 (and particularly 2-20), is particulaly preferable (R: alkyl group).

In the preparing process of the present invention, the mole ratio of alkyl nitrit and carbon monoxide (RONO/CO) in the raw material gas used in the vapor phase catalytic reaction is 2 or more, preferably 2-30, more preferably 2-20, and even more preferably 3-10.

In the present invention, the vapor phase catalytic reaction of alkyl nitrite (RONO) and carbon monoxide (CO) at a mole ratio (RONO/CO) of 2 or more in the presence or a solid catalyst, wherein a platinum group metal ion is exchanged on an ion exchange zeolite carrier, is most characteristic. When the mole ratio of alkyl nitrite and carbon monoxide (RONO/CO) is smaller than 2, the selectivity of the carbonic diester in the above-mentioned vapor phase catalytic reaction becomes no longer adequate, thus making this unsuitable.

In the present invention, the alkyl nitrite and carbon monoxide are preferably supplied to the reaction system either separately or in the form of a mixed gas after diluting with an inert gas such as nitrogen gas. It is also preferable from the viewpoint of safety that the concentration of alkyl nitrite be 30% by volume or less with respect to all of the feed gases (total volume) that are fed in the form of the raw material gas. In addition, it is preferable in terms of productivity on an industrial scale that the concentration of carbon monoxide be 1% by volume or more with respect to all of the feed gases (total volume).

The above-mentioned alkyl nitrite can be obtained in the form of a gas containing alkyl nitrite by reacting nitrogen monoxide, an oxygen-containing gas (for example, air) and a lower alcohol, and the reaction gas can be used directly in the present invention.

Examples of the alkyl nitrite include alkyl nitrites having 1-5 carbon atoms such as methyl nitrite, ethyl nitrite, propyl nitrite and isopropyl nitrite, with methyl nitrite and ethyl nitrite in particular being able to be listed as preferable examples.

In the above-mentioned vapor phase catalytic reaction using a solid catalyst of the present invention, the vapor phase catalytic reaction of alkyl nitrite and carbon monoxide in the presence of moisture at a ratio of 0.01-5 mol% (vol%), and particularly 0.1-3 mol% (vol%), with respect to the total amount (total volume) of raw material gas supplied to the reaction system, is particularly preferable since it allows the activity of the solid catalyst to be maintained at a high level for a long period of time.

In addition, in the above-mentioned vapor phase catalytic reaction of the present invention, the vapor phase catalytic reaction of alkyl nitrite and carbon monoxide in the presence of water at a ratio of 0.01-5 mol% (vol%), and a lower aliphatic alcohol at a ratio of 0.03-30 mol% (vol%), particularly 0.05-20 mol% (vol%), and more particularly 0.1-15 mol% (vol%), with respect to the total amount (total volume) of raw material gas supplied to the reaction system, is preferable.

Examples of the above-mentioned lower aliphatic alcohol include methanol, ethanol, propanol and isopropanol. In addition, in the present invention, the space velocity of all raw material gases (total volume) containing carbon monoxide and alkyl nitrite that are supplied to a reactor filled with solid catalyst is preferably within a range of 500-20000 $h^{-1}$, and particularly preferably within a range of 2000-10000 $h^{-1}$.

In the preparing process of the present invention, the vapor phase catalytic reaction of carbon monoxide and alkyl nitrite can be carried out under extremely mild conditions. For example, it is preferable for said reaction to be carried out at a reaction temperature of 0-200°C, and particularly 50-150°C, and a reaction pressure of atmospheric pressure or under pressurization (particularly at a pressure of roughly 1-20 kg/cm$^2$G).

In the present invention, although the vapor phase catalytic reaction can be carried out in the vapor phase using a batch type or continuous process, a continuous process is industrially advantageous. The form in which the solid catalyst is present in the reaction system is such that the reaction can be carried using either a fixed bed, moving bed or fluid bed for the reactor.

In the present invention, a reaction gas is produced from the reaction system, wherein carbonic diester is formed by the above-mentioned vapor phase catalytic reaction, containing by-products such as oxalic diesters, unreacted carbon monoxide, alkyl nitrites such as methyl nitrite, nitrogen monoxide, carbon dioxide and inert gases in addition to the target product carbonic diester such as dimethyl carbonate.

For example, by cooling the reaction gas wherein dialkyl carbonate has formed in the manner described above, in addition to the target product of dialkyl carbonate, by-products such as dimethyl oxalate are condensed and separated in the form of a reaction liquid. On the other hand, it is preferable that uncondensed gases such as carbon monoxide, methyl nitrite, nitrogen monoxide, carbon monoxide and inert gases be circulated to the reaction system while a portion of those gases is purged.

On the other hand, the reaction liquid (condensed liquid) obtained in the manner described above can be separated and purified by, for example, separating and purifying the carbonic diester such as dimethyl carbonate (the target product) by conventional methods such as distillation.

In addition, the above-mentioned uncondensed gases that are again circulated to the reactor should be added to the raw material gas, should be prepared to a specific amount and should be circulated to the catalytic reaction system. Water and/or a lower aliphatic alcohol may be contained in the circulated gas if necessary.

The solid catalyst used in the preparing process of the present invention is preferably a solid catalyst having weak acidity, wherein a platinum group metal ion, such as palladium, platinum, iridium, ruthenium or rhodium ion, is exchanged on an ion exchange zeolite carrier, and is particularly preferably a solid catalyst having weak acidity wherein at least palladium ion is exchanged on an ion exchange zeolite carrier.

In the above-mentioned solid catalyst, the amount of platinum group metal ion (particularly palladium ion) exchanged on an ion exchange zeolite carrier is the amount wherein the ratio of platinum group metal ion (as platinum group metal ion) to zeolite carrier is preferably 0.1-10% by weight, particularly preferably 0.1-8% by weight, and more particularly preferably 0.5-5% by weight.

The above-mentioned solid catalyst may be a solid catalyst wherein, in addition to platinum group metal ion, metal ion of metals of groups 1b-7b and metals of group 8 (excluding platinum group metals, alkaline metals and alkaline earth metals) of the periodic table, such as copper, iron, tin, nickel, cobalt, silver, cerium or manganese, is exchanged on an ion exchange zeolite carrier so as not to increase the acid site on the carrier. In the present invention, since metal ions of metals of groups 1b-7b and group 8 of the periodic table generally increase the acid site on the carrier, a solid catalyst having weak acidity, wherein only a platinum group metal ion is exchanged on an ion exchange zeolite carrier, is preferable in terms of high space time yield and selectivity of the carbonic diester.

The zeolite carrier preferably used in preparation of the above-mentioned solid catalyst is either an ion exchange zeolite carrier wherein the ion exchange site of the zeolite (or a molecular sieve) is neutralized with alkaline metal ion or alkaline earth metal ion (a powdered zeolite carrier having a particle size of 20-100 μm, or granular zeolite carrier having a grain size of 4-200 mesh), or an ion exchange zeolite carrier wherein the above-mentioned zeolite carrier is further ion exchanged with hydrogen ion and ammonium ion (and particularly, a granular zeolite carrier having a grain size of roughly 5-100 mesh).

Furthermore, a powdered catalyst wherein platinum group metal ion is exchanged on an ion exchange zeolite carrier (powdered) can be used as the above-mentioned solid catalyst. Alternatively, it is preferable that said solid catalyst be used following mixing of a suitable binder into the powdered solid catalyst and molding into granules having a mean grain size of approximately 5-100 mesh.

The "zeolite having an ion exchange site" that serves as the ion exchange zeolite carrier used in preparation of the above-mentioned solid catalyst may be either a synthetic zeolite, such as X zeolite, Y zeolite, Mordenite or silicalite, or a naturally occurring zeolite. The Si to Al atomic ratio of said zeolite is preferably roughly 0.5-10, particularly preferably 1-6, and more particularly preferably 2-5.

In the preparing process of the present invention, the use of a solid catalyst having weak acidity, wherein only palladium ion is exchanged on an "ion exchange zeolite carrier comprising Faujasite zeolite such as X zeolite or Y zeolite", in which the range of the atomic ratio of Si and Al is within roughly 1-10 (preferably 1-6 and more preferably 2-5), is particularly preferable.

A known support method, wherein a platinum group metal compound is added to an aqueous slurry of ion exchange zeolite carrier followed by ion exchange of metal ion on said zeolite carrier, can be used for the method of preparing the above-mentioned solid catalyst. A method for preparing a solid catalyst that can be used industrially is preferable by, for example, adding a complex of a platinum group metal halide, such as tetra-anmine palladium chloride, to an aqueous slurry in which an ion exchange zeolite carrier comprising X zeolite or Y zeolite is dispersed in water, performing ion exchange at a temperature of 5-100°C, and particularly 30-80°C, drying the product in a drying process, and molding with an extruder, tablet machine or granulator as necessary.

Although the above-mentioned drying process does not require any special procedure, drying over a temperature range from room temperature (approximately 20°C) to 100-120°C while slowly raising the temperature over the course of 1-2 hours, and performing drying at that temperature for 1-24 hours, and particularly 2-10 hours, is preferable to form the uniformity of the catalyst.

In addition, in the above-mentioned molding of the solid catalyst, the mixing of a suitable binder into the above-mentioned dried product is preferable in order to improve moldability.

Moreover, in the preparation of the solid catalyst, after exchanging platinum group metal ion on a zeolite carrier and drying, the molded solid catalyst may be baked while flowing in air or an inert gas at a temperature that does not affect the crystal structure or composition of the zeolite carrier (for example, a range of approximately 150-500°C, and preferably a temperature range of 200-350°C) for 1-10 hours.

Examples of the above-mentioned platinum group metal compounds that can be used preferably in the present invention include halides of platinum group metals, inorganic platinum group metallic salts such as nitrates and sulfates, organic platinum group metallic salts such as acetates and oxalates, or various types of complexes such as amine complexes and ethylene diamine complexes of platinum group metal compounds in order to increase solubility in water and so on.

Specific examples of the above-mentioned platinum group metal compounds include palladium compounds such as palladium chlorides (particularly palladium dichloride), palladium acetate and palladium nitrate, the tetra-anmine complexes of those palladium compounds (particularly palladium chlorides), platinum chlorides, tetra-anmine platinum chlorides (complexes), iridium chloride, ruthenium chloride and rhodium chloride.

Moreover, it is particularly preferable that the solid catalyst used in the present invention be a solid catalyst having weak acidity, wherein together with the ion exchange site of an ion exchange zeolite carrier being ion exchange with platinum group metal ion, a portion (at least 10-90% and particularly 20-80%) of the ion exchange site (acid site) on said zeolite carrier other than the location at which platinum group metal ion is supported, is neutralized (or ion exchanged) with alkaline metal ion such as sodium or potassium, or alkaline earth metal ion such as magnesium or calcium.

In the case of the "solid catalyst having weak acidity, wherein platinum group metal ion and alkaline metal ion and/or alkaline earth metal ion are exchanged on the above-mentioned zeolite carrier", since the ion exchange site of the zeolite carrier (referring to an ion exchange site on the above-mentioned zeolite carrier other than that at which platinum group metal ion is exchanged on said zeolite carrier, and is the location of the acid site) is neutralized or exchanged with alkaline metal ion or alkaline earth metal ion, the acid site on the zeolite carrier is reduced considerably, resulting in a decrease in acid strength and acid amount of said resulting solid catalyst.

As a result, in the case of using the above-mentioned solid catalyst having weak acidity in a contact catalytic reaction of alkyl nitrite with carbon monoxide, said solid catalyst offers the advantage of allowing the side reaction rates to be held to a low level.

The acidity of a solid catalyst as in the above-mentioned solid catalyst is expressed according to the acid strength and acid amount. In general, acid strength is determined by a temperature programmed desorption method (TPD method), and is expressed as a relative value to acid amount by TPD method.

In the present invention, the solid catalyst which is used demonstrates weak acid strength having a "desorption temperature of less than 360°C" as indicated with the desorption temperature determined by the ammonia-temperature programmed desorption method (ammonia-TPD method) prescribed by the Catalyst Society.

In particular, the solid catalyst used in the present invention preferably has weak acid strength such that the peak desorption temperature according to the above-mentioned ammonia-TPD method (desorption temperature, an indicator of acid strength) is 350°C or lower (particularly approximately 330°C or lower), and does not essentially demonstrate a peak desorption temperature at approximately 360°C or higher (particularly 350°C or lower). High acidity solid catalysts, which have an acid site that demonstrates excessively strong acid strength, are not preferable since these cause undesirable side reactions in the above-mentioned vapor phase catalytic reaction.

In addition, the "acid amount (total area of each desorption peak by the TPD method)" that relates to the amount of the acid site of the solid catalyst as measured by, for example, the ammonia-TPD method, is 20 or less, particularly 1-10, and preferably 1-6 in the case of taking the "acid amount", relating to the amount of the acid site of HY zeolite measured according to the above-mentioned ammonia-TPD method, to be 100.

Preparation of the above-mentioned "solid catalyst, wherein platinum group metal ion and alkaline metal ion and/or alkaline earth metal ion are exchanged on the above-mentioned zeolite carrier" is preferably performed using a method wherein, for example, after adding alkaline metal compound or alkaline earth metal compound to an aqueous slurry of the above-mentioned ion exchange zeolite carrier and neutralizing the acid site of the above-mentioned zeolite carrier with alkaline metal ion and/or alkaline earth metal ion, platinum group metal compound is added to the aqueous slurry of neutralized zeolite carrier, platinum group metal ion is exchanged on the zeolite carrier and said zeolite carrier is dried followed by molding and baking as necessary.

At the time of the "neutralization procedure" in the above-mentioned preparation of the solid catalyst, the alkaline metal compound or alkaline earth metal compound may be added to the aqueous slurry solution of the zeolite, or an aqueous solution of alkaline metal compound or an aqueous solution of alkaline earth metal compound may be added to the above-mentioned aqueous slurry of the zeolite.

Examples of the above-mentioned alkaline metal compound include salts of organic acids such as sodium acetate, potassium acetate and sodium oxalate, and salts of inorganic acids such as $NaNO_3$, $Na_2SO_4$, NaCl and KCl.
Examples of the above-mentioned alkaline earth metal compound include salts of inorganic acids such as cerium chloride, calcium chloride, magnesium chloride, barium chloride, calcium nitrate, barium nitrate, magnesium nitrate and magnesium phosphate, and salts of organic acids such as calcium acetate and barium acetate.

Although the following provides a detailed explanation of the process of the present invention through its examples and comparative examples, the process of the present invention is not limited to said examples and comparative examples whatsoever.

Furthermore, the space time yield (STY) (g/l·h) in each of the examples and comparative examples was determined from the following equation using "θ" (hr) to represent the contact catalysis reaction time of carbon monoxide and methyl nitrite, "a" (g) to represent the amount of dimethyl carbonate formed during that time, and "b" (l) to represent the amount of catalyst filled into the reaction tube.

$$\text{STY (Space Time Yield)} = a / (b \times \theta)$$

In addition, the selectivities X and Y in each of the examples and comparative examples indicate the selectivity of dimethyl carbonate based on carbon monoxide and based on the methyl nitrite, respectively. Said selectivities X and Y were determined from the following equations using "c" (mol), "d" (mol), "e" (mol), "f" (mol) and "g" (mol) to represent the amounts of dimethyl carbonate, dimethyl oxalate, carbon dioxide, methyl formate and methylal formed during the

above-mentioned time $\theta$ (hr), respectively.

$$X \text{ (Selectivity)} = \{c/(c+2 \times d+e)\} \times 100$$

$$Y \text{ (Selectivity)} = \{c/(c+d+2 \times f+g)\} \times 100$$

Example 1

1) Preparation of Solid Catalyst

10 g of NaY zeolite carrier (having an atomic ratio (Si/Al) of 2.5) were added to 200 ml of distilled water to prepare the zeolite carrier aqueous slurry. Next, 50 ml of an aqueous solution, in which 0.24 g of tetra-anmine palladium chloride (Pd: 1 millimole) is dissolved, were dropped into the above-mentioned aqueous slurry over the course of 30 minutes while stirring and heating said aqueous slurry to 70°C. After stirring while maintaining the temperature at 70°C for 3 hours and ion exchanging the palladium ion on the above-mentioned zeolite carrier, the resulting aqueous slurry was cooled to room temperature followed by filtration and washing with water to obtain a cake-like catalyst.

After placing this wet cake-like catalyst in a drier and drying for 5 hours at 100°C, the dried catalyst was heat treated (drying and baking) for 2 hours at 200°C in the presence of flowing air resulting in the preparing of a solid catalyst wherein palladium ion is exchanged and carried (or loaded) on a Y zeolite carrier.

After molding this solid catalyst with a tablet molding machine and grinding, the resulting particles were passed through a sieve to give the particles a uniform size of 10 mesh to form granules of the solid catalyst.

When the amount of carried palladium ion of the solid catalyst prepared in the manner described above was analyzed using an atomic absorption spectrometer after dissolving with aqua regia, the amount of carried palladium ion was 0.95% by weight, and the amount of carried sodium ion was 8.86% by weight.

In addition, when the acid strength of the above-mentioned solid catalyst was measured according to the ammonia-TPD method, desorption peaks were observed at roughly 195°C and 305°C, and there were essentially no other peaks present at temperatures higher than the above. In addition, the acid amount of the solid catalyst (according to the ammonia-TPD method) was 10% or less of the acid amount of HY zeolite.

2) Preparation of Dimethyl Carbonate

5 ml of the above-mentioned solid catalyst were filled into a reaction tube having an outer diameter of 20 mm (with external jacket), the reaction tube was fixed in a vertical position, heating medium was circulated through the reaction tube jacket, and heating was controlled so that the temperature inside the solid catalyst layer was 120°C.

Raw material gas (having a water content of 0.3 mol% with respect to the entire raw material gas), comprising a composition ratio of 15% by volume of methyl nitrite, 5% by volume of carbon monoxide, 3% by volume of nitrogen monoxide, 2% by volume of methanol and 75% by volume of nitrogen, was fed from the top of the reaction tube at a space velocity (GHSV) of 4000 $h^{-1}$. A vapor phase catalytic reaction was then carried out between methyl nitrite and carbon monoxide for 50 hours under atmospheric pressure while maintaining the reaction temperature at 120°C.

In the above-mentioned vapor phase catalytic reaction, as a result of capturing the reaction gas discharged from the reaction tube from the start of the reaction until 8 hours after the start of the reaction by passing through ice-cooled methanol, and analyzing the captured liquid by gas chromatography, it was confirmed that dimethyl carbonate was formed at an initial space time yield (initial STY) of 390 g/l $\cdot$ h, the initial selectivity of dimethyl carbonate based on carbon monoxide was 85%, and the initial selectivity of dimethyl carbonate based on methyl nitrite was 81%.

In addition, the presence of by-products, in the form of dimethyl oxalate, carbon dioxide, methyl formate and methylal, was also confirmed.

Moreover, the space time yield (STY) and selectivities for the dimethyl carbonate formed in the reaction gas when the above-mentioned vapor phase catalytic reaction was continued for 50 hours remained, essentially unchanged in comparison with the initial space time yield (initial STY) and initial selectivities of dimethyl carbonate (percentage of DMC-STY dropping: within 5%).

Examples 2-4 and Comparative Examples 1-2

With the exception of changing the compositions of methyl nitrite, carbon monoxide and nitrogen gas in the raw material gas, vapor phase catalytic reactions were respectively carried out in the same manner as Example 1 using the solid catalyst prepared in Example 1. The results for "each of the initial space time yields (initial STY) and selectivities of dimethyl carbonate" in these vapor phase catalytic reactions are respectively indicated in Table 1.

Moreover, there were essentially no changes in each of the space time yields and selectivities of dimethyl carbonate when the above-mentioned vapor phase catalytic reactions were continued for 50 hours in these examples in comparison with each of the initial space time yields and selectivities.

On the other hand, in the vapor phase catalytic reaction in Comparative Example 1, in contrast to the initial space time yield (initial STY) of dimethyl carbonate being 130 g/l • h, the space time yield of dimethyl carbonate after 50 hours from the start of the reaction decreased to 60 g/l • h (percentage of DMC-STY dropping: 54%).

Example 5

1) Preparation of Solid Catalyst

With the exception of changing the amount of tetra-anmine palladium chloride to 0.50 g, a solid catalyst, wherein palladium ion is exchanged on the above-mentioned zeolite carrier, was prepared in the same manner as Example 1.

The amount of carried palladium ion of the solid catalyst prepared in the manner described above was 2.07% by weight, while the amount of carried sodium ion was 8.43% by weight.

In addition, the acid strength of the above-mentioned solid catalyst as measured with the ammonia-TPD method demonstrated peak desorption temperatures of approximately 195°C and 305°C, there were essentially no other desorption peaks at temperatures higher than the above. In addition, the acid amount of this solid catalyst was 10% or less of the acid amount of HY zeolite.

2) Preparation of Dimethyl Carbonate

With the exception of using 5 ml of the above-mentioned solid catalyst, a vapor phase catalytic reaction was carried out in the same manner as Example 1. The results for "each of the initial space time yields (initial STY) and selectivities for dimethyl carbonate" in this vapor phase catalytic reaction are respectively indicated in Table 1.

Moreover, there were essentially no changes in the time space yield and selectivities for dimethyl carbonate in comparison with the initial time space yield and selectivities when the above-mentioned vapor phase catalytic reaction was continued for 50 hours in this Example.

Examples 6-7 and Comparative Examples 3-4

1) Preparation of Solid Catalysts

With the exception of using NaX zeolite (Si/Al = 1.23: Example 6) or Na Mordenite (Si/Al = 9.50: Example 7) in place of NaY zeolite, solid catalysts, wherein palladium ion is exchanged on a zeolite carrier, were respectively prepared in the same manner as Example 1.

Each of the amounts of carried palladium of the respective solid catalysts prepared as described above was 0.98% by weight (Example 6 and Comparative Example 3) or 0.92% by weight (Example 7 and Comparative Example 4). When the respective acid strengths were measured by the ammonia-TPD method, desorption peaks were observed at approximately 280°C (Example 6 and Comparative Example 3) and approximately 340°C (Example 7 and Comparative Example 4), respectively. Absorption peaks were essentially not presented at higher temperatures in either case. In addition, although the acid amount of the solid catalyst (according to the ammonia-TPD method) was 10% or less of the acid amount of HY zeolite in Example 6 and Comparative Example 3, it was roughly 15% of the acid amount of HY zeolite in Example 7 and Comparative Example 4.

2) Preparation of Dimethyl Carbonate

With the exception of using each of the above-mentioned solid catalysts, vapor phase catalytic reactions were carried out in the same manner as Example 1 in Examples 6 and 7, and in the same manner as Comparative Example 2 in Comparative Examples 3 and 4. The results of those vapor phase catalytic reactions are respectively indicated in Table 1.

Moreover, the space time yields and selectivities of dimethyl carbonate formed in the reaction gas when each of the above-mentioned vapor phase catalytic reactions were continued for 50 hours in these examples remained essentially unchanged in comparison with the initial space time yield and initial selectivities for dimethyl carbonate, respectively.

Example 8

1) Preparation of Solid Catalyst

With the exception of drying the wet cake-like catalyst for 5 hours at 100°C and heat treating (drying and baking) for 3 hours at 300°C in the presence of flowing air, the granular form of a solid catalyst (grain size: 10 mesh), wherein palladium ion is exchanged on a Y zeolite carrier, was manufactured in the same manner as Example 1.

When the amount of carried palladium ion of the solid catalyst prepared in the manner described above was analyzed with an atomic absorption spectrometer after dissolving with aqua regia, the amount of carried palladium ion was 0.95% by weight, and the amount of carried sodium ion was 8.85% by weight.

In addition, when the acid strength of the above-mentioned solid catalyst was measured with the ammonia-TPD method, desorption peaks were observed at approximately 195°C and 305°C, and there were essentially no desorption peaks present at higher temperatures. In addition, the acid amount (according to the ammonia-TPD method) of the solid catalyst was 10% or less of the acid amount of HY zeolite.

2) Preparation of Dimethyl Carbonate

With the exception of using 2.5 ml of the above-mentioned solid catalyst, using a raw material gas (having a water content of 0.3 mol% with respect to the total amount of raw material gas) composed of 18% by volume of methyl nitrite, 2% by volume of carbon monoxide, 3% by volume of nitrogen monoxide, 2% by volume of methyl alcohol and 75% by volume of nitrogen, supplying said raw material gas to the reaction system at a space velocity of 8000 h$^{-1}$, and controlling heating so that the reaction temperature was 110°C, a vapor phase catalytic reaction was carried out in the same manner as Example 1.

The results for "each of the initial space time yields (initial STY) and selectivities for dimethyl carbonate" in this vapor phase catalytic reaction are indicated in Table 2.

There were essentially no changes in the time space yield and selectivities for dimethyl carbonate in comparison with the initial time space yield and selectivities when the above-mentioned vapor phase catalytic reaction was continued for 50 hours in this example (percentage of DMC-STY dropping: within 5%).

Examples 9-11

With the exception of using the solid catalyst prepared in Example 8 and changing water content of the raw material gas as indicated in Table 2, vapor phase catalytic reactions were respectively carried out in the same manner as Example 8. The results for those vapor phase catalytic reactions are respectively indicated in Table 2.

There were essentially no changes in the space time yields and selectivities with respect to dimethyl carbonate in comparison with the initial space time yields and initial selectivities of dimethyl carbonate when the above-mentioned vapor phase catalytic reactions were respectively continued for 50 minutes in Examples 9 and 10 (percentage of DMC-STY dropping: within 5%).

In addition, in Example 11, the space time yield with respect to dimethyl carbonate formed in the reaction gas when the above-mentioned vapor phase catalytic reactions were respectively continued for 10 hours decreased to 330 g/l·h (percentage of DMC-STY dropping: 13.1%).

Example 12

With the exception of using the solid catalyst prepared in Example 8 and setting the reaction pressure to 3 kg/cm$^2$G, a vapor phase catalytic reaction was carried out in the same manner as Example 8. The results for that vapor phase catalytic reaction are indicated in Table 2.

Example 13

With the exception of using 2.5 ml of the solid catalyst prepared in Example 8, using a raw material gas (having a water content of 0.4 mol% with respect to the entire raw material gas) composed of 18% by volume of methyl nitrite, 3% by volume of carbon monoxide, 3% by volume of nitrogen monoxide, 2% by volume of methanol and 74% by volume of nitrogen, supplying said raw material gas to the reaction system at a space velocity of 8000 h$^{-1}$, controlling heating so that the reaction temperature was 105°C, and setting the reaction pressure to 3 kg/cm$^2$G, a vapor phase catalytic reaction was carried out in the same manner as Example 8.

The results for "each of the initial space time yields (initial STY) and selectivities of dimethyl carbonate" in this vapor phase catalytic reaction are indicated in Table 2.

There were essentially no changes in the time space yield and selectivities for dimethyl carbonate that formed when the above-mentioned vapor phase catalytic reaction was continued for 50 hours in this example in comparison with the initial time space yield and initial selectivities for dimethyl carbonate (percentage of DMC-STY dropping: within 5%).

Examples 14-16

With the exception of the changing the composition of the raw material gas as indicated in Table 2, vapor phase catalytic reactions were respectively carried out in the same manner as Example 13. The results for those vapor phase catalytic reactions are respectively indicated in Table 2.

Example 17

With the exception of changing the reaction pressure to atmospheric pressure, a vapor phase catalytic reaction was carried out in the same manner as Example 13. The results for that vapor phase catalytic reaction are indicated in Table 2.

Example 18

1) Preparation of Solid Catalyst

10.1 g of NaY zeolite having an atomic ratio (Si/Al) of 2.75 (Tosoh Ltd.: HSZ-320NAA) were weighed into a 500 ml beaker followed by the addition of 300 ml of distilled water to prepare an aqueous zeolite slurry. This aqueous slurry was then boiled for 1 hour while stirring with a magnetic stirrer.

300 ml of 1 N $NaNO_3$ were added to the zeolite obtained after filtering this aqueous slurry followed by stirring for 20 hours at room temperature. After filtering this solution and washing with 200 ml of distilled water, an additional 250 ml of distilled water were added to prepare an aqueous zeolite slurry. A solution containing 0.25 g of tetra-anmine palladium chloride dissolved in 50 ml of distilled water was dropped into this slurry while stirring gently over the course of 30 minutes. Moreover, after completion of this dropping and allowing the solution to stand for 1 day while stirring to exchange palladium ion on the zeolite carrier, the aqueous slurry was filtered and washed three times with 200 ml of distilled water.

The resulting aqueous slurry was cooled to room temperature, filtered and washed to obtain a cake-like catalyst. After placing this wet cake-like catalyst in a drier and drying first for 2 hours at 70°C and then 5 hours at 120°C, molding this with a tablet molding machine and grinding, the resulting particles were passed through a sieve to give the particles a uniform size of 10 mesh to form granules of the solid catalyst.

The above-mentioned granules of the solid catalyst were filled into a glass tube equipped with a perforated plate, and heated by raising the temperature from room temperature to 300°C over the course of roughly 1.5 hours while flowing in air at a flow rate of 30 liters/h to bake for 3 hours at that final temperature.

When the amount of carried palladium ion of the solid catalyst prepared in the manner described above was analyzed using an atomic absorption spectrometer after dissolving with aqua regia, the amount of carried palladium ion was 0.95% by weight, and the amount of carried sodium ion was 8.85% by weight.

In addition, when the acid strength of the above-mentioned solid catalyst was measured according to the ammonia-TPD method, desorption peaks were observed at approximately 195°C and 305°C, and there were essentially no other peaks present at temperatures higher than the above. In addition, the acid amount of the solid catalyst (according to the ammonia-TPD method) was 10% or less of the acid amount of HY zeolite.

2) Preparation of Dimethyl Carbonate

After filling 2.5 ml of the above-mentioned solid catalyst into a stainless steel vapor phase reactor having an inner diameter of 10 mm (with external jacket), the reaction tube was fixed in a vertical position, heating medium was circulated through the reaction tube jacket, and heating was controlled so that the temperature inside the solid catalyst layer was 110°C.

A mixed gas of carbon monoxide and a gas containing nitrogen monoxide, oxygen and methyl nitrite synthesized from methanol with nitrogen monoxide (a mixed gas comprising a composition of 15% by volume of methyl nitrite, 5% by volume of carbon monoxide, 3% by volume of nitrogen monoxide, 2% by volume of methanol and 75% by volume of nitrogen (having a water content of 0.05% by volume with respect to the entire raw material gas)), was fed from the top of the reaction tube at a space velocity (GHSV) of 8000 $h^{-1}$. A vapor phase catalytic reaction was then carried out between methyl nitrite and carbon monoxide for 500 hours while maintaining the reaction temperature at 110°C and regulating the pressure of 3 $kg/cm^2G$ at a valve provided at the outlet of the reactor.

In the above-mentioned vapor phase catalytic reaction, as a result of capturing the reaction gas discharged from the reaction tube from the start of the reaction until 8 hours after the start of the reaction by passing through ice-cooled methanol, and analyzing the captured liquid by gas chromatography, it was confirmed that dimethyl carbonate was formed at an initial space time yield (initial STY) of 380 g/l·h, the selectivity of dimethyl carbonate based on carbon monoxide was 88%, and the selectivity of dimethyl carbonate based on methyl nitrite was 81%.

The space time yield and selectivities of the dimethyl carbonate after 70 hours in the above-mentioned vapor phase catalytic reaction remained essentially unchanged in comparison with the initial space time yield (initial STY) and initial selectivities of dimethyl carbonate (percentage of DMC-STY dropping: within 5%)

Moreover, the space time yield of dimethyl carbonate after 500 hours in the above-mentioned vapor phase catalytic reaction was 345 g/l·h (percentage of DMC-STY dropping: 10%).

Example 19

1) Preparation of Solid Catalyst

With the exception of using NaY zeolite having an atomic ratio (Si/Al) of 2.5 (Shokubai Kasei Kogyo Ltd.: ZCE-50), a solid catalyst was prepared, wherein palladium ion is exchanged on a zeolite carrier, in the same manner as Example 18. The solid catalyst prepared in the manner described above had the amount of carried metal ion and properties indicated in Table 3, and the acid amount was nearly the same as the solid catalyst of Example 18.

2) Preparation of Dimethyl Carbonate

With the exception of using the above-mentioned solid catalyst, a vapor phase catalytic reaction was carried out in the same manner as Example 18. The results of that vapor phase catalytic reaction are indicated in Table 3.

Example 20

1) Preparation of Solid Catalyst

With the exception of using NaX zeolite having an atomic ratio (Si/Al) of 1.23 (Union Showa Ltd.: Molecular Sieve 13X), a solid catalyst, wherein palladium ion was exchanged on the above-mentioned zeolite carrier, was prepared in the same manner as Example 18.

The solid catalyst prepared in the manner described above had the amount of carried metal ion and properties indicated in Table 3, and the acid amount was 1.5 times greater than the solid catalyst of Example 18.

2) Preparation of Dimethyl Carbonate

With the exception of using the above-mentioned solid catalyst, a vapor phase catalytic reaction was carried out in the same manner as Example 18. The results of that vapor phase catalytic reaction are indicated in Table 3.

Example 21

1) Preparation of Solid Catalyst

With the exception of changing the amount of tetra-anmine palladium chloride used to 0.50 g, a solid catalyst, wherein palladium ion was exchanged on the above-mentioned zeolite carrier, was prepared in the same manner as Example 18.

The solid catalyst prepared in the manner described above had the amount of carried metal ion and properties indicated in Table 3, and the acid amount was 1.8 times greater than the solid catalyst of Example 18.

2) Preparation of Dimethyl Carbonate

With the exception of using the above-mentioned solid catalyst, a vapor phase catalytic reaction was carried out in the same manner as Example 18. The results of that vapor phase catalytic reaction are indicated in Table 3.

Example 22

1) Preparation of Solid Catalyst

With the exception of using 0.30 g of bis-ethylenediamine palladium chloride instead of 0.25 g of tetra-anmine palladium chloride, a solid catalyst, wherein palladium ion was exchanged on a zeolite carrier, was prepared in the same manner as Example 18.

The solid catalyst prepared in the manner described above had the amount of carried metal ion and properties indicated in Table 3, and the acid amount was nearly the same as that of the solid catalyst of Example 18.

2) Preparation of Dimethyl Carbonate

With the exception of using the above-mentioned solid catalyst, a vapor phase catalytic reaction was carried out in the same manner as Example 18. The results of that vapor phase catalytic reaction are indicated in Table 3.

Example 23

1) Preparation of Solid Catalyst

With the exception of using 300 ml of an aqueous solution of 0.3 N CaCl$_2$ instead of 300 ml of an aqueous solution of 1 N NaNO$_3$, filtering the aqueous slurry, adding the resulting zeolite and stirring for 2 days at room temperature, a solid catalyst, wherein palladium ion and calcium ion were exchanged on a zeolite carrier, was prepared in the same manner as Example 18.

The solid catalyst prepared in the manner described above had the amount of carried metal ion and properties indicated in Table 3, and the acid amount was 1.6 times greater than that of the solid catalyst of Example 18.

2) Preparation of Dimethyl Carbonate

With the exception of using the above-mentioned solid catalyst, a vapor phase catalytic reaction was carried out in the same manner as Example 18. The results of that vapor phase catalytic reaction are indicated in Table 3.

Example 24

1) Preparation of Solid Catalyst

With the exception of using 300 ml of an aqueous solution of 0.3 N LiOAc instead of 300 ml of an aqueous solution of 0.3 N CaCl$_2$, a solid catalyst, wherein palladium and lithium ion are exchanged on a zeolite carrier, was prepared in the same manner as Example 23.

The solid catalyst prepared in the manner described above had the amount of carried metal ion and properties indicated in Table 3, and the acid amount was nearly the same as that of the solid catalyst of Example 18.

2) Preparation of Dimethyl Carbonate

With the exception of using the above-mentioned solid catalyst, a vapor phase catalytic reaction was carried out in the same manner as Example 18. The results of that vapor phase catalytic reaction are indicated in Table 3.

Table 1

| | Kind of zeolite carrier | Characteristic of solid catalyst | | | | | Condition of vapor phase catalytic reaction | | | | | | | Space velocity of raw material gas (h⁻¹) | Reaction temperature (°C) | Reaction pressure kg/cm²G | Reaction time (h) | Result of vapor phase contact reaction | | |
|---|---|---|---|---|---|---|---|---|---|---|---|---|---|---|---|---|---|---|---|---|
| | | Carried amount of metal ion (% by weight) | | | Acid strength: Elimination peak (°C) | Acid amount: against HY type zeolite (%) | Composition of raw material gas (mol %) | | | | | | | | | | | STY of dimethyl carbonate (g/1-h) | Selectivity (%) | |
| | | $Pd^{++}$ | $Na^+$ | Other metals | | | $CH_3ONO$ (MN) | CO | NO | MeOH | $N_2$ | Water | | | | | | | Based on CO | Based on MN |
| Example 1 | Na-Y type zeolite | 0.95 | 8.86 | ——— | 195°C 305°C | 10 or less | 15 | 5 | 3 | 2 | 75 | 0.3 | 4000 | 120 | Atmospheric pressure | 0~8 50 | 390 (same level) | 85 — | 81 — |
| Example 2 | Na-Y type zeolite | 0.95 | 8.86 | ——— | 195°C 305°C | 10 or less | 15 | 3 | 3 | 2 | 77 | 0.3 | 4000 | 120 | Atmospheric pressure | 0~8 50 | 430 (same level) | 85 — | 80 — |
| Example 3 | Na-Y type zeolite | 0.95 | 8.86 | ——— | 195°C 305°C | 10 or less | 15 | 7 | 3 | 2 | 68 | 0.3 | 4000 | 120 | Atmospheric pressure | 0~8 50 | 310 (same level) | 80 — | 73 — |
| Example 4 | Na-Y type zeolite | 0.95 | 8.86 | ——— | 195°C 305°C | 10 or less | 20 | 3 | 3 | 2 | 72 | 0.3 | 4000 | 120 | Atmospheric pressure | 0~8 50 | 490 (same level) | 85 — | 81 — |
| Example 5 | Na-Y type zeolite | 2.07 | 8.43 | ——— | 195°C 305°C | 10 or less | 15 | 3 | 3 | 2 | 77 | 0.3 | 4000 | 120 | Atmospheric pressure | 0~8 50 | 460 (same level) | 85 — | 80 — |
| Example 6 | Na-Y type zeolite | 0.98 | 14.3 | ——— | 280°C | 10 or less | 15 | 5 | 3 | 2 | 75 | 0.3 | 4000 | 120 | Atmospheric pressure | 0~8 50 | 200 (same level) | 85 — | 80 — |
| Example 7 | Na-Y type mordenite | 0.95 | 5.26 | ——— | 340°C | about 15 | 15 | 3 | 3 | 2 | 77 | 0.3 | 4000 | 120 | Atmospheric pressure | 0~8 50 | 120 (same level) | 76 — | 73 — |
| Comparative Example 1 | Na-Y type zeolite | 0.95 | 8.86 | ——— | 195°C 305°C | 10 or less | 10 | 20 | 3 | 2 | 65 | 0.3 | 4000 | 120 | Atmospheric pressure | 0~8 50 | 130 60 | 67 66 | 56 52 |
| Comparative Example 2 | Na-Y type zeolite | 0.95 | 8.86 | ——— | 195°C 305°C | 10 or less | 15 | 15 | 3 | 2 | 65 | 0.3 | 4000 | 120 | Atmospheric pressure | 0~8 50 | 220 140 | 77 71 | 68 60 |
| Comparative Example 3 | Na-Y type zeolite | 0.98 | 14.3 | ——— | 280°C | 10 or less | 15 | 15 | 3 | 2 | 65 | 0.3 | 4000 | 120 | Atmospheric pressure | 0~8 — | 50 — | 72 — | 67 — |
| Comparative Example 4 | Na-Y type mordenite | 0.95 | 5.26 | ——— | 340°C | about 15 | 15 | 15 | 3 | 2 | 65 | 0.3 | 4000 | 120 | Atmospheric pressure | 0~8 — | 30 — | 58 — | 55 — |

EP 0 559 212 B1

Table 2

| | | Characteristic of solid catalyst | | | | | Condition of vapor phase catalytic reaction | | | | | | | | | Result of vapor phase contact reaction | | | |
| | Kind of zeolite carrier | Carried amount of metal ion (% by weight) | | | Acid strength: Elimination peak (°C) | Acid amount: against HY type zeolite (%) | Composition of raw material gas (mol %) | | | | | | Space velocity of raw material gas $(h^{-1})$ | Reaction temperature (°C) | Reaction pressure $kg/cm^2G$ | Reaction time (h) | STY of dimethyl carbonate (g/l-h) | Selectivity (%) | |
| | | $Pd^{++}$ | $Na^+$ | Other metals | | | $CH_3ONO$ (MN) | CO | NO | MeOH | $N_2$ | Water | | | | | | Based on CO | Based on MN |
|---|---|---|---|---|---|---|---|---|---|---|---|---|---|---|---|---|---|---|---|
| Example 8 | Na-Y type zeolite | 0.95 | 8.85 | ——— | 195°C 305°C | 10 or less | 18 | 2 | 3 | 2 | 75 | 0.3 | 8000 | 110 | Atmospheric pressure | 0~8 50 | 280 (same level) | 84 — | 87 — |
| Example 9 | Na-Y type zeolite | 0.95 | 8.85 | ——— | 195°C 305°C | 10 or less | 18 | 2 | 3 | 2 | 75 | 0.5 | 8000 | 110 | Atmospheric pressure | 0~8 50 | 400 (same level) | 83 — | 91 — |
| Example 10 | Na-Y type zeolite | 0.95 | 8.85 | ——— | 195°C 305°C | 10 or less | 18 | 2 | 3 | 2 | 74 | 0.8 | 8000 | 110 | Atmospheric pressure | 0~8 50 | 400 (same level) | 80 — | 85 — |
| Example 11 | Na-Y type zeolite | 0.95 | 8.85 | ——— | 195°C 305°C | 10 or less | 18 | 2 | 3 | 2 | 74 | 1.5 | 8000 | 110 | Atmospheric pressure | 0~8 10 | 380 330 | 75 — | 80 — |
| Example 12 | Na-Y type zeolite | 0.95 | 8.85 | ——— | 195°C 305°C | 10 or less | 18 | 2 | 3 | 2 | 75 | 0.3 | 8000 | 110 | 3 | 0~8 50 | 480 (same level) | 83 — | 90 — |
| Example 13 | Na-Y type zeolite | 0.95 | 8.85 | ——— | 195°C 305°C | 10 or less | 18 | 3 | 3 | 2 | 74 | 0.4 | 8000 | 105 | 3 | 0~8 50 | 400 (same level) | 84 — | 88 — |
| Example 14 | Na-Y type zeolite | 0.95 | 8.85 | ——— | 195°C 305°C | 10 or less | 18 | 3 | 3 | 6 | 70 | 0.4 | 8000 | 105 | 3 | 0~8 50 | 400 (same level) | 81 — | 88 — |
| Example 15 | Na-Y type zeolite | 0.95 | 8.85 | ——— | 195°C 305°C | 10 or less | 18 | 3 | 3 | 12 | 63 | 1.5 | 8000 | 105 | 3 | 0~8 50 | 380 (same level) | 75 — | 87 — |
| Example 16 | Na-Y type zeolite | 0.95 | 8.85 | ——— | 195°C 305°C | 10 or less | 18 | 3 | 3 | 1 | 65 | 0.2 | 8000 | 105 | 3 | 0~8 50 | 380 (same level) | 80 — | 85 — |
| Example 17 | Na-Y type zeolite | 0.95 | 8.85 | ——— | 195°C 305°C | 10 or less | 18 | 3 | 3 | 2 | 74 | 0.4 | 8000 | 105 | Atmospheric pressure | 0~8 50 | 360 (same level) | 83 — | 88 — |

Table 3

| | Characteristic of solid catalyst | | | | | Condition of vapor phase catalytic reaction. | | | | | | | | | | Result of vapor phase contact reaction. | | |
|---|---|---|---|---|---|---|---|---|---|---|---|---|---|---|---|---|---|---|---|
| | Kind of zeolite carrier | Carried amount of metal ion (% by weight) | | | Acid strength: Elimination peak (°C) | Acid amount against HY type zeolite (%) | Composition of raw material gas (mol %) | | | | | | Space velocity of raw material gas (h⁻¹) | Reaction temperature (°C) | Reaction pressure (kg/cm²G) | Reaction time (h) | STY of dimethyl carbonate (g/1-h) | Selectivity (%) | |
| | | Pd | Na | Other metals | | | CH₃ONO (MN) | CO | NO | MeOH | N₂ | Water | | | | | | Based on CO | Based on MN |
| Example 18 | Na-Y type zeolite | 0.95 | 8.86 | --- | 195°C 305°C | 10 or less | 15 | 5 | 3 | 2 | 75 | 0.05 | 8000 | 110 | 3 | 0-8 500 | 380 345 | 88 - | 81 - |
| Example 19 | Na-Y type zeolite | 1.01 | 5.58 | --- | 195°C 315°C | 10 or less | 15 | 5 | 3 | 2 | 75 | 0.05 | 8000 | 110 | 3 | 0-8 70 | 420 409 | 87 - | 80 - |
| Example 20 | Na-Y type zeolite | 0.98 | 14.3 | --- | 190°C 280°C | 10 or less | 15 | 5 | 3 | 2 | 75 | 0.05 | 8000 | 110 | 3 | 0-8 70 | 230 210 | 85 - | 75 - |
| Example 21 | Na-Y type zeolite | 2.07 | 8.43 | --- | 195°C 305°C | 10 or less | 15 | 5 | 3 | 2 | 75 | 0.05 | 8000 | 110 | 3 | 0-8 70 | 480 469 | 86 - | 79 - |
| Example 22 | Na-Y type zeolite | 1.01 | 8.44 | --- | 195°C 305°C | 10 or less | 15 | 5 | 3 | 2 | 75 | 0.05 | 8000 | 110 | 3 | 0-8 70 | 355 348 | 86 - | 9? - |
| Example 23 | Na-Y type zeolite | 1.0 | 6.40 | Ca:4.7 | 196°C 320°C | 10 or less | 15 | 5 | 3 | 2 | 75 | 0.05 | 8000 | 110 | 3 | 0-8 70 | 315 300 | 86 - | 77 - |
| Example 24 | Na-Y type zeolite | 1.0 | 4.80 | Li:1.3 | 194°C 310°C | 10 or less | 15 | 5 | 3 | 2 | 75 | 0.05 | 8000 | 110 | 3 | 0-8 70 | 346 340 | 86 - | 77 - |

The process of the present invention allows the preparation of carbonic diester (dimethyl carbonate) to be carried out for a long period of time at both high selectivity and high yield by carrying out a vapor phase catalytic reaction of a specific ratio of carbon monoxide and alkyl nitrite (e.g., methyl nitrite) in the presence of a solid catalyst wherein plati-

14

num group metal ion is exchanged on an ion exchange carrier such as zeolite.

## Claims

1. A process for preparing a carbonic diester comprising the step of reacting an alkyl nitrite and carbon monoxide in the vapour phase in the presence of a solid catalyst obtainable by ion exchange of a platinum group metal ion on an ion exchange zeolite carrier,
    wherein the mole ratio (RONO/CO) of the alkyl nitrite to carbon monoxide is 2 or more, and wherein the solid catalyst has weak acidity which is characterized by:

    (i) an acidic strength as measured by the desorption temperature determined by the ammonia-temperature programmed desorption (TPD) method prescribed by the Catalyst Society of less than 360°C, and
    (ii) an acidic amount determined by the ammonia-TPD method of 20 or less based upon the acidic amount of an HY type zeolite being 100.

2. A process according to Claim 1, wherein the ion exchange sites of the zeolite carrier at locations other than those at which platinum group metal ions are exchanged, are neutralized or exchanged with alkaline metal ions or alkaline earth metal ions.

3. A process according to Claim 1, wherein the atomic ratio of Si to Al in the zeolite is 0.5 to 10.

4. A process according to Claim 1, wherein said reaction is carried out in the presence of 0.01 to 5 mol % of water based upon the total amount of raw material gas supplied to the reaction system.

5. A process according to Claim 4, wherein the amount of water present is 0.1 to 3 mol % based upon the total amount of raw material gas supplied to the reaction system.

6. A process according to Claim 1, wherein the ratio of the alkyl nitrite to carbon monoxide is 2 to 30.

7. A process according to Claim 6, wherein the ratio of the alkyl nitrite to carbon monoxide is 2 to 20.

8. A process according to Claim 7, wherein the ratio of the alkyl nitrite to carbon monoxide is 3 to 10.

9. A process according to Claim 4, wherein said reaction is carried out in the presence of 0.01 to 5 mol % of water and 0.03 to 30 mol % of a lower aliphatic alcohol based upon the total amount of the raw material gas supplied to the reaction system.

10. A process according to Claim 1, wherein the space velocity of the raw material gas containing carbon monoxide and a nitrite to be reacted is in the range of 500 to 20000 h$^{-1}$.

11. A process according to Claim 1, wherein said reaction is carried out at 0 to 200°C.

12. A process according to Claim 1, wherein the amount of the platinum group metal ions in the catalyst is 0.1 to 10% by weight based on the zeolite carrier.

13. A process according to Claim 1, wherein said ion exchange type zeolite is a synthetic zeolite such as X type zeolite, Y type zeolite, mordenite and silicalite and/or a natural zeolite.

## Patentansprüche

1. Verfahren zur Herstellung eines Kohlensäurediester, das den Schritt des Umsetzens eines Alkylnitrits mit Kohlenmonoxid in der Dampfphase in Gegenwart eines festen Katalysators, der durch Ionenaustausch eines Platingruppenmetallions an einem Zeolith-Ionenaustauscher als Träger erhältlich ist, umfaßt, wobei das Molverhältnis (RONO/CO) Alkylnitrit zu Kohlenmonoxid 2 oder mehr ist, und wobei der feste Katalysator schwache Acidität hat und wie folgt gekennzeichnet ist:

    (i) eine Säurestärke, wie sie durch die Desorptionstemperatur gemessen wird, welche nach dem Verfahren der durch die Ammoniaktemperatur programmierten Desorption (TPD = temperature programmed desorption), das von der Catalyst Society vorgeschrieben ist, bestimmt wird, von weniger als 360 °C, und

(ii) eine Säuremenge, die nach dem Ammoniak-TPD-Verfahren bestimmt wird, von 20 oder weniger, bezogen auf die Säuremenge eines Zeolithen Typ HY, die 100 ist.

2. Verfahren nach Anspruch 1, in dem die Ionenaustauscherstellen des Zeolith als Träger an Stellen, die sich von denen, an die Platingruppenmetallionen ausgetauscht werden, unterscheiden, mit Alkalimetallionen oder Erdalkalimetallionen neutralisiert oder ausgetauscht werden.

3. Verfahren nach Anspruch 1, in dem das Atomverhältnis Si zu Al im Zeolith 0,5 bis 10 ist.

4. Verfahren nach Anspruch 1, in dem die Reaktion in Gegenwart von 0,01 bis 5 mol% Wasser, bezogen auf die Gesamtmenge des gasförmigen Ausgangsmaterials, das dem Reaktionssystem zugeführt wird, durchgeführt wird.

5. Verfahren nach Anspruch 4, in dem die vorliegende Wassermenge 0,1 bis 3 mol%, bezogen auf die Gesamtmenge des gasförmigen Ausgangsmaterials, das dem Reaktionssystem zugeführt wird, beträgt.

6. Verfahren nach Anspruch 1, in dem das Verhältnis Alkylnitrit zu Kohlenmonoxid 2 bis 30 ist.

7. Verfahren nach Anspruch 6, in dem das Verhältnis Alkylnitrit zu Kohlenmonoxid 2 bis 20 ist.

8. Verfahren nach Anspruch 7, in dem das Verhältnis Alkylnitrit zu Kohlenmonoxid 3 bis 10 ist.

9. Verfahren nach Anspruch 4, in dem die Reaktion in Gegenwart von 0,01 bis 5 mol% Wasser und 0,03 bis 30 mol% eines niedrigeren aliphatischen Alkohols, bezogen auf die Gesamtmenge des gasförmigen Ausgangsmaterials, das dem Reaktionssystem zugeführt wird, durchgeführt wird.

10. Verfahren nach Anspruch 1, in dem die Raumgeschwindigkeit des gasförmigen Ausgangsmaterials, das Kohlenmonoxid und ein Alkylnitrit, die umgesetzt werden sollen, enthält, im Bereich von 500 bis 20 000 $h^{-1}$ liegt.

11. Verfahren nach Anspruch 1, in dem die Reaktion bei 0 bis 200 °C durchgeführt wird.

12. Verfahren nach Anspruch 1, in dem die Menge des Platingruppenmetallions in dem Katalysator 0,1 bis 10 Gew.%, bezogen auf den Träger Zeolith, ist.

13. Verfahren nach Anspruch 1, in dem der Zeolith-Ionenaustauscher ein synthetischer Zeolith Typ Y, Mordenit und Silicalite und/oder ein natürlicher Zeolith ist.

## Revendications

1. Procédé de préparation d'un diester carbonique, comprenant l'étape consistant à faire réagir un nitrite d'alkyle et du monoxyde de carbone en phase vapeur, en présence d'un catalyseur solide susceptible d'être obtenu par échange d'un ion d'un métal du groupe du platine sur un support zéolithique d'échange d'ions,
dans lequel le rapport molaire (RONO/CO) du nitrite d'alkyle au monoxyde de carbone est de 2 ou plus, et
dans lequel le catalyseur solide possède une faible acidité qui est caractérisée par:

(i) une force acide, mesurée par la température de désorption, déterminée par la méthode de désorption à l'ammoniac et à programme de température (TPD) décrite par la Catalyst Society, inférieure à 360°C, et
(ii) une quantité acide, déterminée par la méthode ammoniac/TPD, de 20 ou moins, rapportée à la valeur 100 attribuée à la quantité acide d'une zéolithe de type HY.

2. Procédé selon la revendication 1, dans lequel les sites d'échange d'ions du support zéolithique aux endroits autres que ceux auxquels sont échangés les ions du métal du groupe du platine, sont neutralisés ou échangés par des ions de métaux alcalins ou des ions de métaux alcalino-terreux.

3. Procédé selon la revendication 1, dans lequel le rapport atomique de Si à Al, dans la zéolithe, est de 0,5 à 10.

4. Procédé selon la revendication 1, dans lequel ladite réaction est réalisée en présence de 0,01 à 5% moles d'eau par rapport à la quantité totale de matière première gazeuse introduite dans le système réactionnel.

5. Procédé selon la revendication 4, dans lequel la quantité d'eau présente est de 0,1 à 3% moles, par rapport à la quantité totale de matière première gazeuse introduite dans le système réactionnel.

6. Procédé selon la revendication 1, dans lequel le rapport du nitrite d'alkyle au monoxyde de carbone est de 2 à 30.

7. Procédé selon la revendication 6, dans lequel le rapport du nitrite d'alkyle au monoxyde de carbone est de 2 à 20.

8. Procédé selon la revendication 7, dans lequel le rapport du nitrite d'alkyle au monoxyde de carbone est de 3 à 10.

9. Procédé selon la revendication 4, dans lequel ladite réaction est réalisée en présence de 0,01 à 5% moles d'eau et de 0,03 à 30% moles d'un alcool aliphatique inférieur, par rapport à la quantité totale de matière première gazeuse introduite dans le système réactionnel.

10. Procédé selon la revendication 1, dans lequel la vitesse spatiale de la matière première gazeuse contenant du monoxyde de carbone et un nitrite devant réagir, est dans la gamme de 500 à 20 000 $h^{-1}$.

11. Procédé selon la revendication 1, dans lequel ladite réaction est réalisée à une température de 0 à 200°C.

12. Procédé selon la revendication 1, dans lequel la quantité d'ions du métal du groupe du platine dans le catalyseur est de 0,1 à 10% en poids, par rapport au support zéolithique.

13. Procédé selon la revendication 1, dans lequel ladite zéolithe du type à échange d'ions est une zéolithe synthétique telle qu'une zéolithe du type X, une zéolithe du type Y, une mordénite et une silicalite et/ou une zéolithe naturelle.